**Europäisches Patentamt**

**European Patent Office**

**Office européen des brevets**

(19)

(11) Veröffentlichungsnummer: **0 011 190**
**B1**

(12) **EUROPÄISCHE PATENTSCHRIFT**

(45) Veröffentlichungstag der Patentschrift: **16.02.83**

(21) Anmeldenummer: **79104253.4**

(22) Anmeldetag: **02.11.79**

(51) Int. Cl.³: **C 08 F 20/10,**
**C 08 F 20/20, C 08 F 2/44**
**//A61K6/08**

(54) Füllstoffhaltige, vernetzte Perlpolymerisate.

(30) Priorität: **14.11.78 DE 2849279**
**17.11.78 DE 2849936**

(43) Veröffentlichungstag der Anmeldung:
**28.05.80 Patentblatt 80/11**

(45) Bekanntmachung des Hinweises auf die Patenterteilung:
**16.02.83 Patentblatt 83/7**

(84) Benannte Vertragsstaaten:
**AT CH DE FR GB IT**

(56) Entgegenhaltungen:
**EP - A - 0 011 186**
**NL - A - 7 103 123**
**US - A - 3 069 375**

(73) Patentinhaber: **BAYER AG**
**Zentralbereich Patente, Marken und Lizenzen**
**D-5090 Leverkusen 1, Bayerwerk (DE)**

(72) Erfinder: **Podszun, Wolfgang, Dr.**
**Wolfskaul 4**
**D-5000 Köln 80 (DE)**
Erfinder: **Süling, Carlhans, Dr.**
**Carl-Leverkus-Strasse 10**
**D-5068 Odenthal (DE)**
Erfinder: **Walkowiak, Michael, Dr.**
**Albertus-Magnus-Strasse 10**
**D-5090 Leverkusen 1 (DE)**
Erfinder: **Schulz, Hans-Hermann**
**Am Neulandkreuz 23**
**D-5653 Leichlingen 1 (DE)**

Courier Press, Leamington Spa, England.

# 0011190

## Füllstoffhaltige, vernetzte Perlpolymerisate

Gegenstand der Erfindung sind mit anorganischem Füllstoff gefüllte, vernetzte Perlpolymerisate mit einem mittleren Perlendurchmesser ($d_{50}$) von 5 bis 500 $\mu$m auf Basis von (Meth)-acrylsäureestern.

Die Herstellung von ungefüllten Perlpolymerisaten durch Perlpolymerisation ist z.B. aus Houben-Weyl, Methoden der Organischen Chemie, Bd. XIV/1 bekannt. Man suspendiert dazu das oder die Monomeren in Wasser unter Rühren und polymerisiert die gebildeten Tröpfchen mithilfe eines monomerlöslichen Initiators.

Die Perlpolymerisation erfordert im allgemeinen Dispergatoren. Diese verhindern ein Verkleben der Perlen währen der Reaktion. Sie sind in der Regel wasserlösliche, hochmolekulare organische Verbindungen oder feinteilige, wasserunlösliche anorganische Verbindungen; letztere werden auch als Pickering-Dispergatoren bezeichnet. Viele verschiedene Substanzen werden als Pickering-Dispergator verwendet, z.B. die Carbonate, Phosphate, Sulfate und Silikate der Erdalkalimetalle, Aluminiumoxid und Aluminiumhydroxid, Bentonit und Talkum.

Dabei werden die Körnchen des Dispergiermittels nicht im Inneren der Perlen eingeschlossen, sondern befinden sich praktisch ausschließlich auf der Perlenoberfläche, so daß sie bei der Aufarbeitung leicht entfernt werden können. Auf diese Weise können Perlpolymerisate von hoher Reinheit gewonnen werden.

In einigen Fällen ist bereits versucht worden, Farbstoffe und Pigmente in Perlpolymerisate einzupolymerisieren. So ist aus der US—PS 2 533 196 bekannt, pigmentierte Polymerisatperlen aus Dicarbonsäurediallylester herzustellen. Beim Arbeiten gemäß der US—PS 2 533 196 wird der monomere Dicarbonsäurediallylester anpolymerisiert und mit einem Pigment versetzt; die angedickte pigmentierte Masse wird in einer wäßrigen Natrium-bisulfit- oder Natriumnitrat-Lösung suspendiert und unter Rühren ausgehärtet. Das Dispergiermedium muß bei diesem Verfahren die gleiche Dichte wie die anpolymerisierte pigmentierte Masse besitzen.

Des weiteren wird in der DE—PS 829 221 ein Verfahren zur Herstellung von leuchtfarbenhaltigen Polymerisationskunstoffen beschrieben, bei dem die Suspensionspolymerisation in Gegenwart von Leuchtfarben und einem Verdickungsmittel durchgeführt wird.

Die US—PS 2 533 196 und die DE—PS 829 221 nutzen die Tatsache aus, daß hochviskose Flüssigkeiten eingearbeitete Feststoffe stärker festhalten als weniger viskose Flüssigkeiten.

Die Schwierigkeiten der Perlpolymerisation von hochviskosen Substanzen sind bekannt: die gebildeten Flüssigkeitströpfchen neigen in verstärktem Maße zur Agglomeration und es werden überwiegend große, d.h. ein bis einige Millimeter große Perlen gebildet (siehe dazu auch FR—PS 1 005 601). Bei der Perlpolymerisation von mit anorganischem Füllstoff gefüllten Polymer/Monomermischungen beeinträchtigt die erhöhte Sedimentationstendenz der gefüllten Flüssigkeitströpfchen die Ausbildung von relativ kleinen, runden Polymerisatperlen zusätzlich. Es ist daher nicht möglich, durch Arbeiten in Analogie zu der US—PS 2 533 196 oder DE—PS 829 221 mit anorganischem Füllstoff gefüllte Perlen mit einem mittleren Perlendurchmesser unter 1 mm herzustellen. Bei entsprechenden Versuchen werden Teilchen von mehreren Millimetern Durchmesser erhalten, deren Form stark von der Kugelform abweicht.

Gegenstand der Niederländischen Patentanmeldung 7 103 123 ist die Herstellung von Tunern für Photokopierverfahren. Bei den Materialien handelt es sich unter anderem um Perlpolymerisate, die bestimmte Pigmente enthalten. Es wird jedoch nichts über die Verwendung hochviskoser Monomere, die Mitverwendung von Vernetzungsmitteln oder mögliche Anwendungen in der Dentaltechnik ausgesagt.

Gegenstand der Erfindung sind demgegenüber vernetzte Perlpolymerisate mit einem mittleren Perlendurchmesser ($d_{50}$) von 5—500 $\mu$m, bestehend aus polymerisierten (Meth)-acrylsäureestern und gegebenenfalls bis zu 20 Gewichtsprozent anderen Monomeren, die einen anorganischen, feinteiligen Füllstoff in gleichmäßig verteilter Form enthalten. Die erfindungsgemäßen Perlpolymerisate sind dadurch charakterisiert, daß sie durch folgende Verfahrensschritte hergestellt worden sind:

a) Der anorganische Füllstoff wird in eine Monomerflüssigkeit oder eine Lösung von Polymerisat in Monomerflüssigkeit eingearbeitet, wobei das Monomer bzw. die Lösung eine Viskosität von 0,1 bis 10 Pa.s, gemessen bei der Dispergiertemperatur, besitzt und die Monomerflüssigkeit ein Vernetzungsmittel enthält;

b) Die Mischung aus Füllstoff, Monomerflüssigkeit und gegebenenfalls Polymerisat wird mit einem Polymerisationsinitiator versetzt und die Mischung in einem wäßrigen Medium in Gegenwart eines Dispergators suspendiert;

c) Schließlich wird die Suspension durch Erhitzen auf die Zerfallstemperatur des Initiators polymerisiert.

Die erfindungsgemäßen gefüllten Perlpolymerisate eignen sich als Füllmittel in pastenförmigen Dentalmassen; sie werden leicht durch die flüssige Pastenkomponente (Monomer) benetzt und zeigen nach der Aushärtung eine gute mechanische Verankerung in der Kunststoffmatrix.

Der Vernetzeranteil, bezogen auf Monomerflüssigkeit bzw. Lösung von Polymerisat in Monomerflüssigkeit, kann bei der Herstellung der erfindungsgemäßen Perlpolymerisate im Falle des Ausführungsbeispiels 5 bei nur 4 Gew.-% Ethylenglycoldimethacrylat liegen; es können jedoch auch im Falle der Ausführungsbeispiele 6 und 7 100 Gew.-% des Umsetzungsprodukts aus Trimethylhexamethylendiiso-

2

cyanat und 2-Hydroxyethylmethacrylat als Vernetzer verwendet werden.

Bei der Herstellung der erfindungsgemäßen Perlpolymerisate wird zunächst in einem ersten Arbeitsschritt der anorganische Füllstoff unter kräftigem Rühren in eine viskose Monomerflüssigkeit oder eine viskose Mischung aus Monomerflüssigkeit und Polymerisat eingearbeitet. Unter "viskos" ist dabei zu verstehen, daß die Monomerflüssigkeit bzw. die Mischung eine Viskosität von 0,1 bis 10 Pa.s besitzen soll. Die Zahlenangaben gelten für die Temperatur, bei der die Masse dispergiert wird. Bei Viskositäten unter 0,1 Pa s findet kein oder nur ein mengenmäßig sehr geringer Einschluß der anorganischen Partikel bei der nachfolgenden Polymerisation statt; bei höheren Viskositäten als 10 Pa s können keine kleinen Perlen erhalten werden, d. h., eine definierte Viskosität der Monomerphase ist für den technischen Effekt Voraussetzung.

Als Monomere können die aliphatischen Ester der Methacrylsäure und Acrylsäure, wie beispielsweise Methyl(meth)acrylat, Ethyl(meth)acrylat oder Cyclohexylmethacrylat verwendet werden, daneben als Vernetzungsmittel Methacrylsäureester von mehrwertigen Alkoholen, wie Ethylenglykoldimethacrylat, Tetraethylenglykoldimethacrylat und Trimethylolpropantrimethacrylat. Neben (Meth)acrylaten können auch bis zu 20 Gew.-% andere Monomere, wie Stryol, Divinylbenzol oder Vinylacetat eingesetzt werden.

Bei Verwendung dieser Monomeren muß im allgemeinen die Viskosität durch teilweise Polymerisation oder durch Zugabe von im Monomer löslichen, getrennt hergestellten Polymerisat erhöht werden.

Man kann auch Monomere einsetzen, deren Viskosität hoch genug ist. Beispiele hierfür sind das Bis-GMA (Umsetzungsprodukt von Bisphenol A und Glycidylmethacrylat) oder durch Addition von Diisocyanaten und Hydroxialkyl(meth)acrylaten entstandene Urethan(meth)acrylate.

Als Füllstoffe eignen sich in Wasser unlösliche, feinteilige anorganische Substanzen, z.B. Metalloxide, -sulfate, -silikate und -phosphate, sowie Gläser und keramische Massen und deren Mischungen. Der Korndurchmesser der Füllstoffe ist im allgemeinen unter 5$\mu$m, vorzugsweise unter 2$\mu$m.

Im zweiten Arbeitsschritt wird die mit anorganischem Füllstoff gefüllte viskose Mischung mit einer Initiatorsubstanz (z.B. Benzoylperoxid, Cyclohexylpercarbonat) versetzt und durch hochtouriges Rühren in einer wäßrigen Lösung eines z.B. hochmolekularen Dispergators suspendiert. Dabei hat die Dispergatorlösung im allgemeinen einen Dispergatorgehalt von 0,2 bis 5 Gew.-%. Der Suspendiervorgang erfolgt im allgemeinen bei Raumtemperatur (20 bis 25°C). Doch läßt sich in manchen Fällen durchaus der negative Temperaturkoeffizient der Viskosität in dem Sinne ausnutzen, daß Mischungen, deren Viskosität bei Raumtemperatur zu hoch (d.h., höher als 100 Pa s) ist, bei erhöhter Temperatur suspendiert werden.

Als Dispergatoren eignen sich besonders gut durch partielle Verseifung gewonnene Copolymere von Vinylalkohol/Vinylacetat oder durch Copolymerisation hergestellte Methacrylsäure/Methacrylsäuremethylester-Copolymerisate.

Im dritten Arbeitsschritt wird die Suspension unter Rühren durch Erhitzen auf die Zerfallstemperatur der Initiatorsubstanz polymerisiert. Dabei ist ein Aufheizen unter Druck von Vorteil, um besonders regelmäßige Perlen zu erhalten.

Aus der auspolymerisierten Suspension kann auf bekannte Weise durch Filtrieren, Waschen und Trocknen das Perlpolymerisat gewonnen werden.

Die in den Beispielen angegebenen Viskositäten wurden bei 25°C mit einem Kugelfallviskosimeter bestimmt. Die Werte gelten jeweils für die Monomerphase in Abwesenheit des anorganischen Füllstoffs.

Beispiel 1
Herstellung eines mit Magnesiumhydroxidcarbonat gefüllten Perlpolymerisats.

Reaktionsgefäß:
3-Liter-Planschliffbecher mit Blattrührer, Rückflußkühler, Innenthermometer, Gaseinlaß- und Gasauslaßrohr.

Mischung 1: Monomerphase

| | | |
|---|---|---|
| 220 g | Methylmethacrylat | Viskosität |
| 30 g | Ethylenglykoldimethacrylat | der Mischung: |
| 50 g | Polymethylmethacrylat | 0,6 Pa s |
| | ([$\eta$] = 1,05 in Chloroform) | |

100 g Magnesiumhydroxidcarbonat
(4 MgCO$_2$.Mg(OH)$_2$. 4 H$_2$O)
(Hersteller: Riedel de Haen AG)
Vernetzeranteil in Monomerflüssigkeit + Polymerisat: 10 Gew.-%

Mischung 2: wäßrige Phase

15 g Molviol ® 70/88 (teilverseiftes Polyvinylacetat mit einer Verseifungszahl von 88 der Farbwerke Hoechst AG), gelöst in 1000 ml destilliertem Wasser.

Die Komponenten der Mischung 1 werden unter Ausschluß von Luftsauerstoff in das Reaktionsgefäß gegeben und 12 Std. bei Raumtemperatur mit 250 UpM gerührt, wobei das Polymerisat gelöst wird und eine hochviskose Masse gebildet wird. Diese Mischung wird mit 2,2 g Cyclohexylpercarbonat versetzt und weitere 30 Min. gerührt. Danach wird die Mischung 2 auf einmal zugegeben und die Rührgeschwindigkeit auf 800 UpM erhöht. Man erhitzt die gebildete Suspension auf 70°C und kühlt bei einsetzender exothermer Reaktion so stark, daß die Temperatur unter 85°C gehalten wird. Nach dem Abklingen der Reaktion wird der Ansatz unter weiterem Rühren 2 Std. lang auf 85°C gehalten. Das feste Perlpolymerisat wird nach dem Abkühlen abfiltriert, mehrfach mit dest. Wasser gewaschen und bei 50°C getrocknet.

Ausbeute: 328 g
mittlere Korngröße: 160 $\mu$m.

0,5 g des Perlpolymerisats werden 30 Min. lang bei 70°C mit 20 ml 1n-Salzsäure behandelt, anschließend mit dest. Wasser gewaschen und getrocknet. Durch diesen Vorgang wird nicht fest in den Perlen eingeschlossenes Magnesiumhydroxidcarbonat entfernt. Nach der Salzsäurebehandlung beträgt der Magnesiumhydroxidcarbonatanteil der Perlen 14,1 Gew.-%.

Beispiel 2 (Vergleichsbeispiel)
Perlpolymerisation *ohne* Einbau von Magnesiumhydroxidcarbonat.

Reaktionsgefäß: wie in Beispiel 1

Mischung 1: Monomerphase

270 g Methylmethacrylat ⎱ Viskosität
30 g Ethylenglykoldimethacrylat ⎰ der Mischung: 0,001 Pa s

100 g Magnesiumhydroxidcarbonat
(4 $MgCO_3.Mg(OH)_2.4 H_2O$)
(Hersteller: Riedel de Haen AG)
Vernetzeranteil in Monomerflüssigkeit: 10 Gew.-%

Mischung 2: wäßrige Phase wie in Beispiel 1

Mischung 1 wird unter Luftausschluß in das Reaktionsgefäß gegeben und unter langsamem Rühren mit 2,7 g Cyclohexylpercabonat versetzt. Danach wird Mischung 2 auf einmal zugegeben und die Rührgeschwindigkeit auf 800 UpM eingestellt. Das weitere Vorgehen erfolgt wie in Beispiel 1. Man erhält 277 g Perlpolymerisat mit einer mittleren Korngröße von 42 $\mu$m. Das Perlpolymerisat enthält nach der in Beispiel 1 beschriebenen Salzsäurebehandlung *kein* Magnesiumhydroxidcarbonat. (Der analytische Fehler ist kleiner als 0,05%).

Beispiel 3
Herstellung eines mit Titandioxid gefüllten Perlpolymerisats

Reaktionsgefäß wie in Beispiel 1

Mischung 1: Monomerphase

225 g Methylmethacrylat ⎱ Viskosität
15 g Ethylenglykoldimethacrylat ⎰ der Mischung:
60 g Polymethylmethacrylat ⎰ 1,5 Pa s
([$\eta$] = 1,05 in Chloroform)

150 g Titandioxid
(Bayer AG)
Vernetzeranteil in Monomerflüssigkeit/Polymerisat: 5 Gew.-%

Mischung 2: wäßrige Phase
700 ml dest. Wasser
300 ml MMA—MAS-Dispergatorlösung
[7,5%ige wäßrige Lösung eines Copolymerisats aus gleichen Gewichtsteilen Methacrylsäure und Methylmethacrylat mit pH = 6 (mit NaOH eingestellt) und der Viskosität von 3,6 Pa s.]

Die Komponenten der Mischung 1 werden unter Ausschluß von Luftsauerstoff in das Reaktionsgefäß gegeben und 12 Std. bei Raumtemperatur mit 250 UpM gerührt, wobei das Polymerisat gelöst wird und eine hochviskose Masse gebildet wird.

Diese Mischung wird mit 1,5 g Lauroylperoxid und 1,5 g Benzoylperoxid versetzt und weitere 30 Min. gerührt. Danach wird die Mischung 2 auf einmal zugegeben und die Rührgeschwindigkeit auf 800 UpM erhöht. Man erhitzt die gebildete Suspension auf 80°C und kühlt bei einsetzender exothermer Reaktion so stark, daß die Temperatur unter 88°C gehalten wird. Nach dem Abklingen der Reaktion wird der Ansatz unter weiterem Rühren 2 Std. lang auf 85°C gehalten. Das feste Perlpolymerisat wird nach dem Abkühlen durch Abdekantieren von feinteiligen Anteilen befreit, anschließend filtriert, mehrfach mit dest. Wasser gewaschen und bei 50°C getrocknet.

Ausbeute: 407 g
Verbrennungsrückstand: 32,2%

Siebanalyse:
>200 $\mu$m 7,02%
100 — 200 $\mu$m 48,76%
63 — 100 $\mu$m 26,32%
40 — 63 $\mu$m 11,06%
0 — 40 $\mu$m 6,84%

Abb. 1

Aufnahmen im Hellfeld
M 199/1—2
V = 133:1
(0,50 mm ≙ 66,5 mm Papier)

Abb. 2

Hellfeld
M 199/17—18
V = 133:1
(0,5 mm ≙ 66,5 mm Papier)

Abbildung 1 zeigt die Fraktion 40 bis 63 $\mu$m in einer mikroskopischen Aufnahme im Hellfeld bei einem Maßstab von 133:1. Die Perlen erscheinen dunkel infolge der starken Lichtstreuung der $TiO_2$-Partikel. Der Füllstoff ist gleichmäßig verteilt.

Abbildung 2 zeigt zum Vergleich eine Aufnahme eines ungefüllten Perlpolymerisats gemäß Beispiel 2, ebenfalls als Hellfeldaufnahme bei einem Maßstab von 133:1.

Beispiel 4
Herstellung eines mit Bariumsulfat gefüllten Perlpolymerisats.

Reaktionsgefäß wie in Beispiel 1

Mischung 1: Monomerphase
220 g Methylmethacrylat $\Big\}$ Viskosität
30 g Ethylenglykoldimethacrylat $\Big\}$ der Mischung:
50 g Polymethylmethacrylat $\Big\}$ 0,6 Pa s
($[\eta] = 1,05$ in Chloroform)

100 g Bariumsulfat
(Riedel de Haen AG)
Vernetzeranteil in Monomerflüssigkeit + Polymerisat: 10 Gew.-%

Mischung 2: wäßrige Phase wie in Beispiel 3

Die Komponenten der Mischung 1 werden unter Ausschluß von Luftsauerstoff in das Reaktionsgefäß gegeben und so lange bei Raumteperatur verrührt, bis das Polymerisat gelöst ist. Die erhaltene Mischung wird mit 1,1 g Lauroylperoxid und 1,1 g Benzoylperoxid versetzt und, wie im Beispiel 3 beschrieben, polymerisiert und aufgearbeitet.

Ausbeute: 375 g
Verbrennungsrückstand: 28,8%.

Siebanalyse:
$>200\ \mu$m 2,38%
$100 - 200\ \mu$m 2,44%
$63 - 100\ \mu$m 13,92%
$40 - 63\ \mu$m 38,06%
$0 - 40\ \mu$m 43,20%

Beispiel 5
Herstellung eines mit Zirkondioxid gefüllten Perlpolymerisats.

Reaktionsgefäß: 6-Liter-Autoklav mit Doppelankerrührer

Mischung 1: Monomerphase
580 g Methylmethacrylat $\Big\}$ Viskosität
30 g Ethylenglykoldimethacrylat $\Big\}$ der Mischung:
110 g Polymethylmethacrylat $\Big\}$ 6 Pa s
($[\eta]$ in Chloroform = 2,0)

100 g Zirkondioxid (E. Merck, Darmstadt)
Vernetzeranteil in Monomerflüssigkeit + Polymerisat: 4 Gew.-%

Mischung 2: wäßrige Phase
2100 ml dest. Wasser
900 ml MMA—MAS-Dispergatorlösung, wie in Beispiel 3 beschrieben.

Die Komponenten der Mischung 1 werden unter Luftausschluß in den Autoklaven gegeben und 12 Std. mit 100 UpM bei Raumtemperatur gerührt. Diese Mischung wird mit 3 g Lauroylperoxid und 3 g Benzoylperoxid versetzt und weitere 30 Min. gerührt. Nach der Zugabe von Mischung 2 wird 15 bar Stickstoff aufgedrückt, die Rührgeschwindigkeit auf 450 UpM eingestellt und auf 80°C aufgeheizt. Bei einsetzender Reaktion wird so stark gekühlt, daß die Temperatur unter 90°C gehalten wird. Man läßt den Ansatz 2 Std. lang bei 80°C nachrühren. Das Perlpolymerisat wird nach dem Abkühlen abfiltriert, mehrfach mit dest. Wasser gewaschen und bei 50°C getrocknet.

Ausbeute: 802 g
Verbrennungsrückstand: 12,5%.

Siebanalyse:
```
        >200 µm 26,00%
100 — 200 µm 30,24%
 63 — 100 µm 21,20%
 40 —  63 µm 13,22%
  0 —  40 µm  9,34%
```

### Beispiel 6
Herstellung eines mit Kieselsäure gefüllten Perlpolymerisats.

Reaktionsgefäß: wie in Beispiel 1

Mischung 1: Monomerphase
```
188 g  Bisphenol-A-bis-(3-methacrylato-2-hydroxypropyl)ether ⎱ Viskosität der
112 g  Triethylenglykoldimethacrylat                          ⎰ Mischung:
 33 g  hochdisperse Kieselsäure                                  ca.1 Pa s
       (BET—Oberfläche 170 m²/g)
Vernetzeranteil in Monomerflüssigkeit: 100 Gew.-%
```

Mischung 2: wäßrige Phase
```
700 ml  dest. Wasser
300 ml  MMA—MAS-Dispergatorlösung, wie. in Beispiel 3 beschrieben.
```

Mischung 1 wird mit 3,0 g Benzoylperoxid versetzt und, wie in Beispiel 3 beschrieben, polymerisiert.

Ausbeute: 305 g
Verbrennungsrückstand: 9,5%

Siebanalyse:
```
        >100 µm  0,68%
 63 — 100 µm  7,56%
 40 —  63 µm 34,16%
  0 —  40 µm 57,60%
```

### Beispiel 7
Herstellung eines mit Kieselsäure und Bariumsulfat gefüllten Perlpolymerisats.

Reaktionsgefäß: wie in Beispiel 1

Mischung 1:
```
300 g  Plex ® 6661 der Firma Röhm, Darmstadt (Umsetzungsprodukt aus Trimethylhexamethyl-
       endiisocyanat und 2-Hydroxyethylmethacrylat), Viskosität 7,5 Pa s.
 30 g  hochdisperse Kieselsäure.
       (BET-Oberfläche 170 m²/g)
100 g  Bariumsulfat
       (Riedel de Haen)
Vernetzeranteil in Monomerflüssigkeit: 100 Gew.-%
```

Mischung 2:
```
700 ml  dest. Wasser
300 ml  MMA—MAS-Dispergatorlösung, wie in Beispiel 3 beschrieben.
```

Mischung 1 wird mit 5 g Cyclohexylpercarbonat versetzt und, wie in Beispiel 3 beschrieben, polymerisiert.

Ausbeute: 385 g
Verbrennungsrückstand: 25,5%.

Siebanalyse:
```
        >200 µm 28,46%
100 — 200 µm 46,82%
 63 — 100 µm 16,32%
 40 —  63 µm  5,52%
  0 —  40 µm  2,88%
```

7

**0 011 190**

**Patentansprüche**

1. Vernetzte Polymerisatperlen mit einem mittleren Perlendurchmesser ($d_{50}$) von 5 bis 500 $\mu$m, bestehend aus polymerisierten (Meth)acrylsäureestern und gegebenenfalls bis zu 20 Gewichtsprozent anderen Monomeren, enthaltend einen anorganischen, feinteiligen Füllstoff in gleichmäßig verteilter Form, dadurch gekennzeichnet, daß sie hergestellt worden sind, in dem man

a) den anorganischen Füllstoff in eine Monomerflüssigkeit oder eine Lösung von Polymerisat in Monomerflüssigkeit einarbeitet, wobei das Monomer bzw. die Lösung eine Viskosität von 0,1 bis 10 Pa.s, gemessen bei der Dispergiertemperatur, besitzt,

b) der Mischung aus Füllstoff, Monomerflüssigkeit und gegebenenfalls Polymerisat einen Polymerisationsinitiator zusetzt und die Mischung in einem wäßrigen Medium in Gegenwart eines Dispergators suspendiert und

c) die Suspension durch Erhitzen auf die Zerfallstemperatur des Initiators polymerisiert, wobei die Monomerflüssigkeit ein Vernetzungsmittel enthält.

2. Polymerisatperlen nach Anspruch 1, dadurch gekennzeichnet, daß sie hergestellt worden sind unter Verwendung einer Lösung eines Polymerisats in der Monomerflüssigkeit.

3. Polymerisatperlen nach Anspruch 2, dadurch gekennzeichnet, daß die Lösung des Polymerisats in der Monomerflüssigkeit durch teilweise Polymerisation des Monomeren hergestellt worden ist.

4. Polymerisatperlen nach Anspruch 1 bis 3, dadurch gekennzeichnet, daß sie unter Verwendung von Methacrylsäureestern mehrwertiger Alkohole als Vernetzungsmittel hergestellt worden sind.

5. Polymerisatperlen nach Anspruch 1 oder 4, dadurch gekennzeichnet, daß sie unter Verwendung von Bis-GMA oder Additionsprodukten von Diisocyanaten und Hydroxyalkyl(meth)acrylaten als Monomer hergestellt worden sind.

**Revendications**

1. Perles de polymères réticulés à un diamètre moyen de perle ($d_{50}$) de 5 à 500$\mu$m, consistant en esters (méth)acryliques polymérisés et le cas échéant jusqu'à 20% en poids d'autres monomères, contenant une matière de charge minérale en fines particules à l'état uniformément répartie, caractérisées en ce qu'elles ont été préparées de la manière suivante:

a) on incorpore la matière de charge minérale dans un liquide monomère ou dans une solution de polymère dans un liquide monomère, le monomère ou la solution présentant une viscosité de 0,1 à 10 Pa.s mesurée à la température de dispersion,

b) au mélange de la matière de charge, du liquide monomère et le cas échéant du polymère, on ajoute un inducteur de polymérisation et on met le mélange en suspension dans un milieu aqueux en présence d'un agent dispersant, et

c) on polymérise la suspension par chauffage à la température de décomposition de l'inducteur, le liquide monomère contenant un agent réticulant.

2. Perles de polymères selon la revendication 1, caractérisées en ce qu'elles ont été préparées par utilisation d'une solution d'un polymère dans le liquide monomère.

3. Perles de polymères selon la revendication 2, caractérisées en ce que la solution du polymère dans le liquide monomère a été préparée par polymérisation partielle du monomère.

4. Perles de polymères selon les revendications 1 à 3, caractérisées en ce qu'elles ont été préparées par utilisation d'esters méthacryliques d'alcools polyvalents en tant qu'agents réticulants.

5. Perles de polymères selon la revendication 1 ou 4, caractérisées en ce qu'elles ont été préparées par utilisation du bis-GMA ou de produits d'addition de diisocyanates et de (méth)acrylates d'hydroxyalkyle en tant que monomère.

**Claims**

1. Cross-linked polymer beads having an average bead diameter ($d_{50}$) of 5 to 500 $\mu$m, consisting of polymerised (meth)acrylic acid esters and optionally up to 20 per cent by weight of other monomers, containing an inorganic finely divided filler in a uniformly distributed form, characterised in that they have been prepared by

a) working the inorganic filler into a monomer liquid or a solution of polymer in monomer liquid, the monomer or the solution having a viscosity of 0.1 to 10 Pa.s, measured at the dispersion temperature,

b) adding a polymerisation initiator to the mixture of filler, monomer liquid and if appropriate polymer and by suspending the mixture in an aqueous medium in the presence of a dispersant and

c) polymerising the suspension by heating it to the decomposition temperature of the initiator, wherein the monomer liquid contains a cross-linking agent.

2. Polymer beads according to Claim 1, characterised in that they have been produced using a solution of a polymer in the monomer liquid.

3. Polymer beads according to Claim 2, characterised in that the solution of the polymer in the monomer liquid has been prepared by partial polymerisation of the monomer.

4. Polymer beads according to Claim 1 to 3, characterised in that they have been produced using methacrylic acid esters of polyhydric alcohols as cross-linking agents.

5. Polymer beads according to Claim 1 or 4, characterised in that they have been produced using bis-GMA or addition products of diisocyanates and hydroxyalkyl (meth)acrylates as the monomer.